# EUROPEAN PATENT SPECIFICATION

(11) **EP 0 887 051 B1**
(45) Date of publication and mention of the grant of the patent: **17.04.2002**
(21) Application number: 98304961.0
(22) Date of filing: 24.06.1998
(51) Int. Cl.: A61F 2/06

(54) **Stent with reinforcing struts and bimodal deployment**
Stent mit verstärkenden Streben und bimodaler Entfaltung
Stent avec supports renforcés et à déploiement bimodale

(30) Priority: 24.06.1997 US 881059
(43) Date of publication of application: 30.12.1998
(73) Proprietor: Advanced Cardiovascular Systems, Inc., Santa Clara, CA 95054-8167 (US)
(72) Inventor: Allen, Richard T., Palo Alto, California 94306 (US); Cox, Daniel L., Palo Alto, California 94301 (US)
(74) Representative: McLeish, Nicholas Alistair Maxwell

(56) References cited:
- EP-A- 0 679 373
- EP-A- 0 758 541

## Description

### BACKGROUND OF THE INVENTION

The present invention relates to expandable endoprosthesis devices, generally called stents, which are adapted to be implanted into a body lumen of a patient, such as a blood vessel, to maintain the patency thereof. These devices are useful in the treatment and repair of atherosclerotic stenoses in blood vessels.

Stents are generally cylindrically-shaped devices which function to hold open and sometimes to expand a segment of a blood vessel or other anatomical lumen. Stents particularly are suitable for use to support and to hold back a dissected arterial lining which, if not so supported and held, can occlude the fluid passageway therethrough.

A variety of devices are known in the art for use as stents and have included: coiled wires in an array of patterns that are expanded after having been placed intraluminally via a balloon catheter; helically-wound coiled springs manufactured from an expandable heat-sensitive metal; and self-expanding stents inserted in a compressed state and shaped in a zigzag pattern. Additional examples of prior art stents are shown in U.S. Patent No. 4,776,337 to Palmaz; U.S. Patent No. 4,655,771 to Wallsten; U.S. Patent No. 4,800,882 to Gianturco; U.S. Patent No. 4,913,141 to Hillstead; and U.S. Patent No. 5,292,331 to Boneau.

In EP-A-0679373, a stent is disclosed for implanting in a body lumen and expanding from a contracted condition to an expanded condition. The stent comprises a plurality of adjacent cylindrical elements arranged in alignment along a longitudinal stent axis, each cylindrical element being independently expandable in a radial direction and formed in an irregular serpentine wave pattern transverse to the longitudinal axis and containing a plurality of alternating peaks and valleys, and at least one interconnecting member extending between adjacent cylindrical elements and connecting them to one another. The stent further comprises an anti-thrombogenic coating covering the cylindrical elements and the interconnecting members.

Prior art devices include an expandable intraluminal vascular graft that is expanded within a blood vessel by a balloon which typically is associated with a dilatation catheter. The graft may be a wire mesh tube, a stainless steel tube with rectangular openings, or a tube with honeycomb style openings. Another prior art device includes a prosthesis for transluminal implantation comprising a flexible tubular body made of flexible thread elements wound together, each thread having a helix configuration.

There are still more conventional endovascular stents. In one design, the wire stent has a generally cylindrical shape, wherein the shape is formed with alternating bent wire loops. Another conventional stent design comprises a series of continuous corrugations compressed together to form a tube-like mesh. Yet another endovascular stent used for the treatment of restenosis is a unitary wire structure, shaped to criss-cross to form a plurality of upper and lower peaks.

One of the difficulties encountered using prior art stents involved being able to maintain the radial rigidity needed to hold open a body lumen while at the same time maintaining the longitudinal flexibility of the stent, so as to facilitate its delivery. Another difficulty was the limited range of expansion available. Certain prior art stents expanded only to a limited degree due to the uneven stresses created during radial expansion. This made it necessary to provide stents having a variety of diameters, thus increasing the cost of manufacture. Having access to stents with various expanded diameters also allowed the physician to be in a position to re-dilate the stent if the original vessel size had been miscalculated.

Another difficulty with the prior art stents was that the stent contracted along its longitudinal axis when the device was expanded radially. This made it difficult to place the stent within the artery during expansion.

Various means have been devised to deliver and implant stents. One method frequently described for delivering a stent to a desired intraluminal location involved mounting the expandable stent on an expandable member, such as an inflatable balloon, which was provided on the distal end of an intravascular catheter. The catheter was advanced to the desired location within the patient's body lumen. Inflating the balloon on the catheter deformed the stent to a permanently expanded condition. The balloon then was deflated and the catheter removed.

What has been needed and heretofore unavailable is a stent which has a high degree of flexibility so that it can be advanced through tortuous passageways leading to the desired deployment site and can be radially expanded over a wide range of diameters with minimal longitudinal contraction, and yet have the mechanical strength to hold open the body lumen into which it is expanded. There is further a need for a stent that has high circumferential or "hoop" strength to improve crush resistance.

### SUMMARY OF THE INVENTION

Accordingly, the present invention provides a longitudinally flexible stent as defined in claim 1.

The present invention is directed to an expandable stent having a configuration generally of the type disclosed in U.S. Patent Nos. 5, 569,295 to S. Lam and 5,514,154 to Lau et al. According to the invention, the stent includes a plurality of adjacent cylindrical elements which are expandable in the radial direction and which are arranged in alignment along a longitudinal stent axis. The cylindrical elements are formed in a serpentine wave pattern transverse to the longitudinal axis and contain a plurality of alternating peaks and valleys.

The stent also comprises at least one interconnecting member that extends between adjacent cylindrical elements and connects adjacent cylindrical elements to each other. The interconnecting members insure minimal longitudinal contraction of the stent during radial expansion of the cylindrical elements.

The stent further comprises, in each cylindrical element, a reinforcing member that extends across each peak and valley. More precisely, each peak and each valley of a single cylindrical element is formed by the confluence of two straight struts joining at a bend. The reinforcing member thus spans across the peak or valley, bridging the struts.

The reinforcing member lends strength to the alternating peaks and valleys, wherein the area of maximum stress is at or near the bend. To be sure, the reinforcing member prevents the straight section of the strut from buckling or distorting during expansion of the stent by adding material to a potentially weak area. Further, the size and geometry of the reinforcing member along with the bend may be adjusted so that stress is evenly distributed between the two instead of just being borne by the bend.

Certainly the geometry of the reinforcing member can assume many configurations. For example, the reinforcing member can include a loop that curves toward or away from the bend. The reinforcing member can join the struts at a point farther away from or closer to the bend. The reinforcing member can be formed into the bend.

The resulting stent structure preferably is a series of radially-expandable cylindrical elements that are spaced longitudinally closely enough to each other so that the elements will press back into position small dissections in the wall of a body lumen, but not so closely as to compromise the longitudinal flexibility of the stent. The individual cylindrical elements may rotate slightly relative to adjacent cylindrical elements without significant deformation, cumulatively providing a stent which is flexible along its length and about its longitudinal axis, but which is still very stable in the radial direction and thus resistant to collapse.

A stent embodying features of the present invention can be readily delivered to the desired lumenal location by mounting it on an expandable member of a delivery catheter, for example a balloon, and by then passing the catheter-stent assembly through the body lumen to the implantation site. A variety of means for securing the stent to the expandable member on the catheter for delivery to the desired location are available. It presently is preferred to compress the stent onto the balloon. Other means to secure the stent to the balloon include providing ridges or collars on the inflatable member to restrain lateral movement, or using temporary, bioabsorbable adhesives.

The stent by use of the reinforcing members features bimodal deployment. That is, when the stent is expanded radially as described above, it does so in two stages. In the first stage, the struts bend slightly outwardly, to accommodate the increasing circumference of each cylindrical element, and the loop portion of the reinforcing member is stretched out. The second stage continues from the first stage with the struts continuing to bend outwardly, the most severe bending occurring at the reinforcing member, until the point at which the struts are pulled apart as widely as possible to accommodate the largest diameter that the stent can assume. Further spreading of the struts is prevented by the presence of the reinforcing member, which limits the maximum circumference of each cylindrical element. By choosing the size and geometry of the reinforcing member and the struts, the amount of force needed to expand the stent to a particular diameter can be altered.

The cylindrical elements of the stent preferably are plastically deformed when expanded (except when nickel-titanium (NiTi) alloys are used as the material from which the elements are formed) so that the stent remains in the expanded condition. Therefore, when non-NiTi elements are used, the elements must be sufficiently rigid following expansion so as to prevent the elements from collapsing after deployment. With super-elastic NiTi alloys, the expansion occurs when the stress of compression is removed as relief from compression causes the phase transformation of the material from the martensite phase back to the expanded austenite phase.

After the stent is expanded, some of the peaks and/or valleys may tip outwardly and become embedded in the vessel wall. Thus, after expansion, the stent does not have a smooth outer wall surface, but rather is characterized by projections which embed in the vessel wall and thus aid in retaining the stent in place in the vessel.

Other features and advantages of the present invention will become more apparent from the following detailed description when taken in conjunction with the accompanying exemplary drawings.

### BRIEF DESCRIPTION OF THE DRAWINGS

FIG. 1 is an elevational view, partially in section, depicting a stent which is mounted on a delivery catheter and disposed within a body lumen such as a coronary artery.

FIG. 2 is an elevational view, partially in section, similar to that shown in FIG. 1, wherein the stent is expanded within the artery, pressing the dissected lining against the arterial wall.

FIG. 3 is an elevational view, partially in section, showing the expanded stent within the vessel after withdrawal of the delivery catheter.

FIG. 4 is an enlarged partial view of the stent of FIG. 5 depicting a serpentine pattern having peaks and valleys that form the cylindrical elements of the stent.

FIG. 5 is a plan view of a flattened section of a stent which illustrates the serpentine pattern of the stent.

FIG. 6 is a side elevational view of the stent in the expanded condition.

FIGS. 7A-L are top plan views of alternative examples of a single reinforced peak or valley.

FIG. 8 is a plan view of an embodiment of the present invention reinforced stent.

FIG. 9 illustrates an alternative embodiment of the present invention reinforced stent.

FIGS. 10A and B show the bimodal deployment of a stent.

### DETAILED DESCRIPTION OF THE PREFERRED EMBODIMENTS

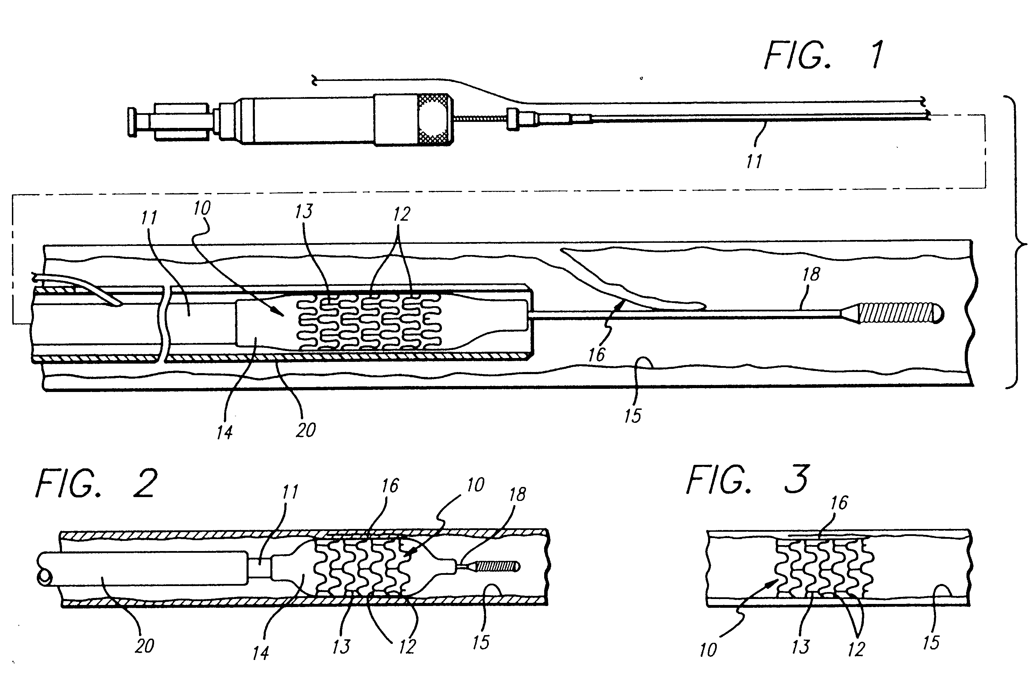

FIG. 1 illustrates a stent 10 which is mounted onto a delivery catheter 11. The stent 10 generally comprises a plurality of radially expandable cylindrical elements 12 disposed coaxially and interconnected by members 13 disposed between adjacent cylindrical elements. The delivery catheter 11 has an expandable portion or balloon 14 for expanding stent 10 within an artery 15 or other vessel. The artery 15, as shown in FIG. 1, has a dissected lining 16 which has occluded a portion of the arterial passageway.

The delivery catheter 11 onto which stent 10 is mounted is essentially the same as the balloon dilatation catheter that conventionally is used for angioplasty procedures such as percutaneous transluminal angioplasty (PTA) or percutaneous transluminal coronary angioplasty (PTCA). The balloon 14 may be formed of suitable materials such as polyethylene, polyethylene terephthalate, polyvinyl chloride, nylon and ionomers such as those manufactured under the trademark SURLYN by the Polymer Products Division of the E.I. duPont deNemours Company. Other polymers also may be used. In order for the stent 10 to remain in place on the balloon 14 during delivery to the site of the damage within the artery 15, the stent 10 is compressed onto the balloon. An elastic protective sheath sometimes is attached around the balloon 14 so that the stent 10 is crimped onto the sheath, which protects the balloon from the metal stent 10 and insures uniform expansion of the stent when the balloon and elastic sheath are expanded. A retractable protective delivery sleeve 20 also may be provided to further insure that the stent stays in place on the expandable portion of the delivery catheter 11 and to prevent abrasion of the body lumen by the open or outer surface of the stent 10 during delivery to the desired arterial location. Other means for securing the stent 10 onto the balloon 14 also may be used, such as providing collars or ridges on the ends of the working portion, i.e., the cylindrical portion, of the balloon.

Each radially expandable cylindrical element 12 of stent 10 may be independently expanded. Therefore, the balloon 14 may be provided with an inflated shape other than cylindrical, e.g., tapered, to facilitate implantation of the stent 10 in a variety of body lumen shapes.

The delivery of the stent 10 is accomplished in the following manner. The stent 10 first is mounted onto the inflatable balloon 14 on the distal extremity of the delivery catheter 11. The stent may be "crimped" down onto the balloon to insure a low profile. The catheter-and-stent assembly can be introduced within the patient's vasculature with a conventional Seldinger technique through a guiding catheter (not shown). A guide wire 18 is disposed across the arterial section with the detached or dissected lining 16 and then the catheter-stent assembly is advanced over the guide wire 18 within the artery 15 until the stent 10 is positioned within the artery at the detached lining 16. The balloon 14 of the catheter is expanded, expanding the stent 10 against the artery 15, which is illustrated in FIG. 2. While not shown in the drawing, the artery 15 preferably can be expanded slightly by the expansion of stent 10 to seat or otherwise fix the stent 10 to prevent movement within the artery. In some circumstances during the treatment of stenotic portions of an artery, the artery may have to be expanded considerably in order to facilitate passage of blood or other fluid therethrough.

The stent 10 serves to hold open the artery 15 after the catheter 11 is withdrawn, as illustrated in FIG. 3. Due to the formation of the stent 10 from an elongated tubular member, the undulating component of the cylindrical elements of the stent 10 is relatively flat in transverse cross-section, so that when the stent is expanded, the cylindrical elements are pressed into the wall of the artery 15 and, as a result, the risk of development of thrombosis in the artery 15 is minimized. The cylindrical elements 12 of the stent 10, which are pressed into the wall of the artery 15, eventually will be covered with endothelial cell growth which also discourages thrombosis. The serpentine pattern of the cylindrical sections 12 provide good tacking characteristics to prevent stent movement within the artery. Further, the closely spaced cylindrical elements 12 at regular intervals provide uniform support for the wall of the artery 15, and consequently are well adapted to tack up and to hold in place small flaps or dissections in the wall of the artery 15, as illustrated in FIGS. 2 and 3.

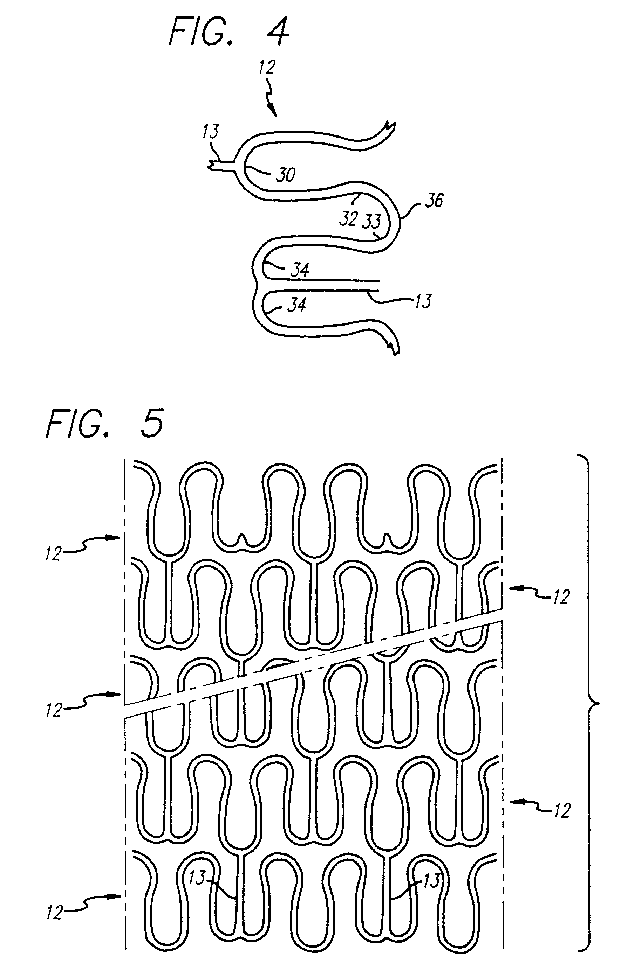

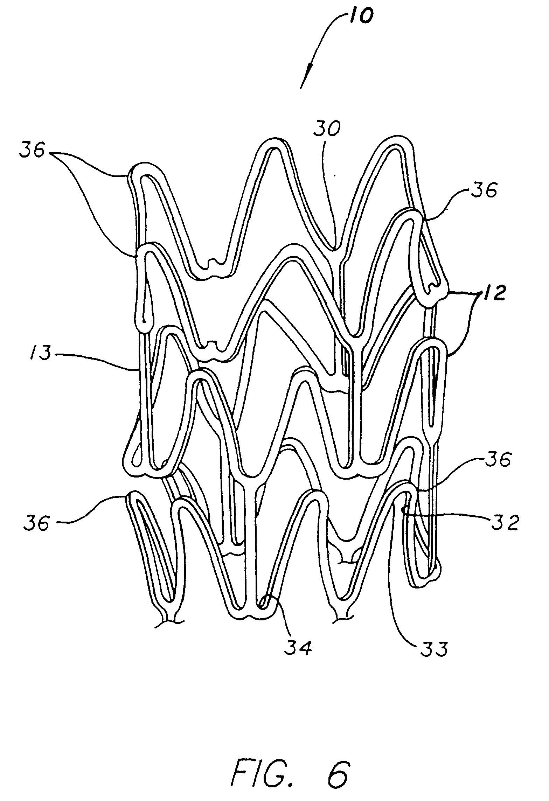

As depicted in FIGS. 4, 5 and 6, the stresses involved during expansion from a low profile to an expanded profile are much more evenly distributed among the various peaks 36 and valleys 34 than was experienced with other stents. As seen in FIG. 4, a portion of a cylindrical element 12 of the stent 10, illustrates the serpentine pattern having a plurality of peaks 36 and valleys 34, each characterized by varying radii of curvature 30, 32, 33, which varying radii of curvature aid in the even distribution of expansion forces. The interconnecting members 13 serve to connect adjacent valleys 34 of the cylindrical elements 12 as described above.

After expansion, portions of the various elements will turn outwardly, forming small projections which will embed in the vessel wall. For example, the tip of a peak portion 36 tips outwardly when it has been expanded to a degree that is sufficient to embed the tip into the vessel wall and thereby to help secure the implanted stent. Upon expansion, the projecting peak 36 provides an outer wall surface on the stent that is not smooth, but instead has a plurality of projecting peaks 36 all along the outer wall surface. While the projections assist in securing the stent in the vessel wall, they are not sharp and thus do not cause trauma or damage to the vessel wall.

One important feature is the capacity of the stent to expand from a low-profile delivery diameter to a diameter much greater than heretofore could be achieved, while still maintaining structural integrity of the stent in the expanded state. Due to its novel structure, the stent of the present invention has an overall expansion ratio of 1 up to about 4 using certain compositions of stainless steel. For example, a 316L stainless steel stent of the present invention can be radially expanded from a diameter of 1 unit up to a diameter of about 4 units, which deforms the structural members beyond their elastic limits. The stent still retains its structural integrity in the expanded state and it serves to hold open the vessel in which it is implanted. Materials other than 316L stainless steel may give higher or lower expansion ratios without sacrificing structural integrity.

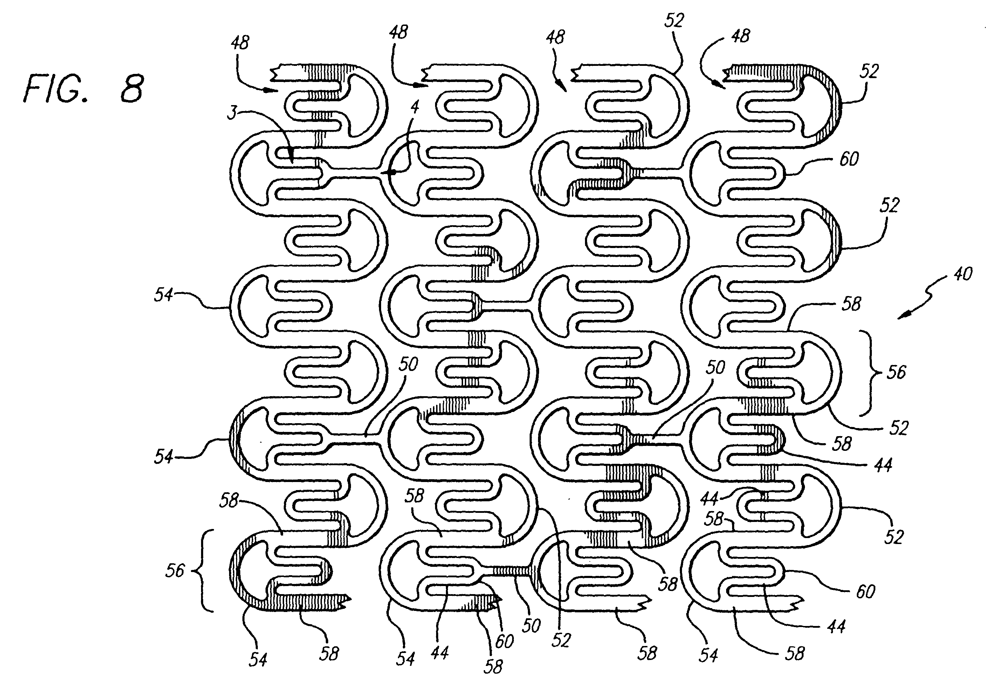

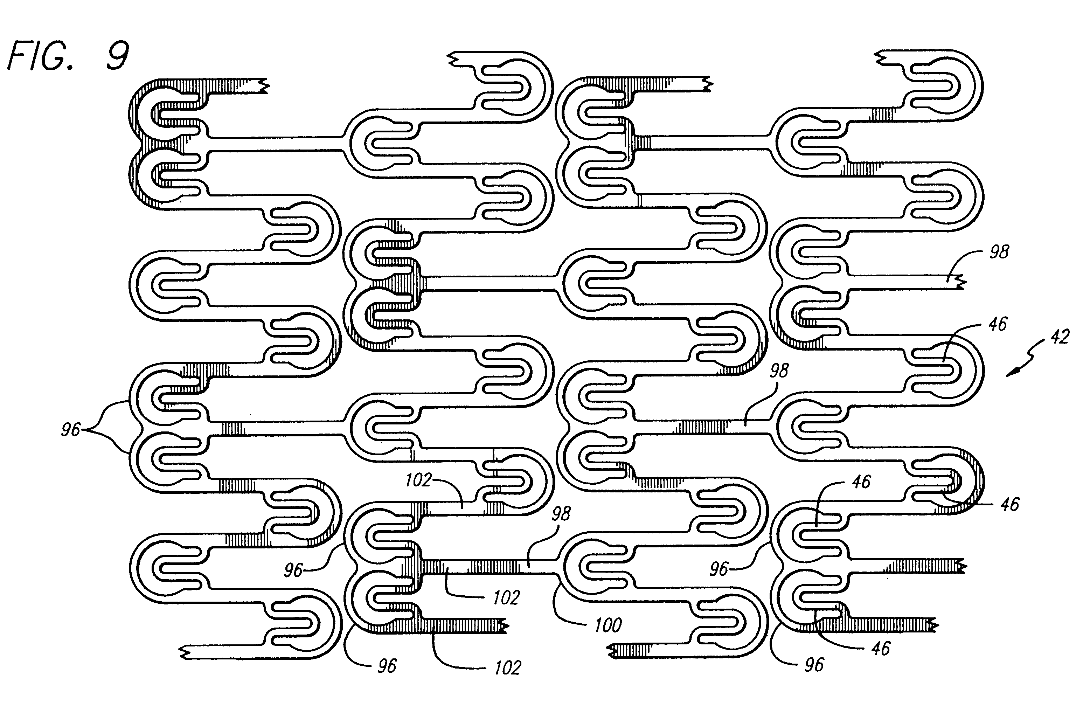

FIGS. 8 and 9 are plan views of examples of stents embodying the invention, each illustrating a flattened section of a stent 40 and a stent 42. The FIGS. illustrate the serpentine patterns of the stents as well as varying configurations of the reinforcing members 44, 46. In the preferred embodiment illustrated in FIG. 8, the stent 40 is comprised of a plurality of radially expandable cylindrical elements 48, which are disposed generally coaxially and interconnected by interconnecting members 50.

As in the earlier described stents the presently preferred embodiment shown in FIG. 8 includes alternating peak portions 52 and valley portions 54. Each peak portion 52 or valley portion 54 is essentially a bend 56 interconnecting straight struts 58. In this embodiment, each peak portion 52 or valley portion 54 is reinforced by a reinforcing member 44 extending across the bend 56 to the interconnecting struts 58. In the preferred embodiment depicted in FIG. 8, the reinforcing member 44 has an inverted loop 60 that extends in a direction opposite to bend 56. Optionally, the interconnecting members 98 may be integrated into the loops 60 of the reinforcing members 46, as illustrated in FIG. 9.

The area of peak stress is at or near the apex of each bend 56.

This area is reinforced with the reinforcing members 44, each of which is attached to each side of a bend (i.e., to the struts 58) away from the apex of the bend 56. The width of the struts 58, along with the width and geometry of the reinforcing members 44 and the geometry and dimensions of the bends 56 (which form either peak portions 52 or valley portions 54) can be adjusted to distribute the stress between the bends 56 and the reinforcing members 44. Further, varying the base material from which the stent is formed will affect the design of the bends 56 and the reinforcing members 44.

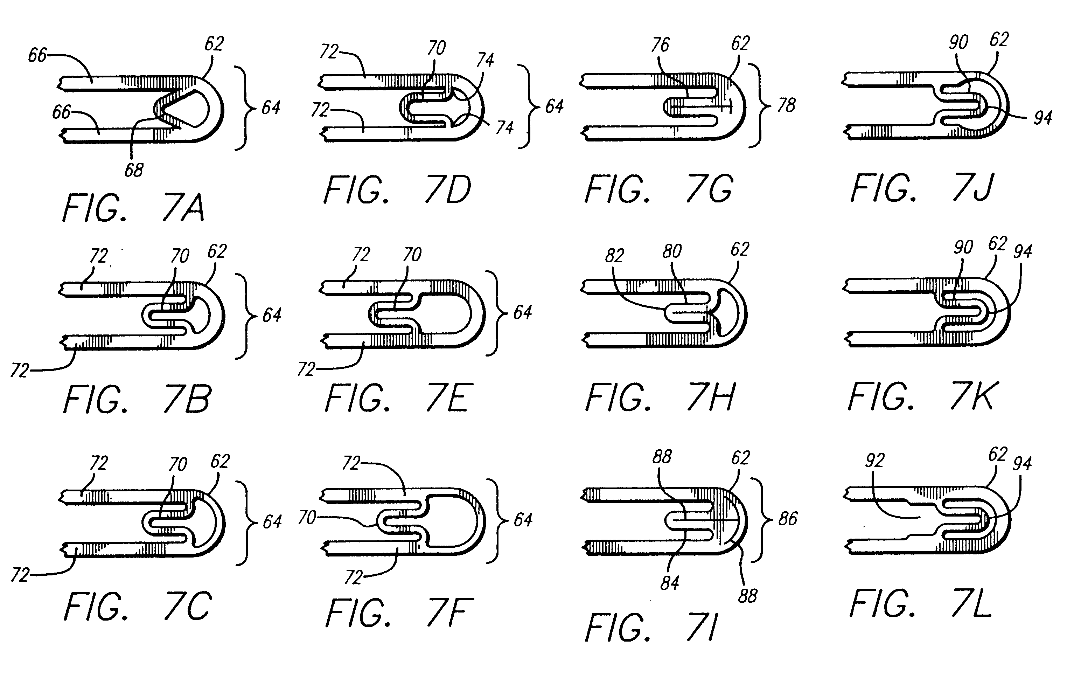

FIG. 7 illustrates a variety of alternative structures what may comprise either the peak portions or the valley portions of a stent embodying the invention. Specifically, reinforcing members of different constructions are shown in plan views. As seen in FIG. 7A, the peak or valley portion 62 is formed by a bend 64 which is supported by struts 66. The reinforcing member 68 has a V-shape and is integrated into the bend 64. FIGS. 7B and 7C show peak or valley portions 62 with different thicknesses for the bends 64. FIG. 7D illustrates a reinforcing member 70 that intersects the struts 72, wherein the point of intersection creates sharpened corners 74 that are rounded in FIGS. 7B, 7C, 7D, 7E, and 7F. In FIGS. 7E and 7F, the reinforcing member 70 has been moved further down the struts 72, i.e., farther away from the bend 64. FIG. 7G depicts an alternative structure wherein the reinforcing member 76 has been integrated into the bend 78. In FIG. 7H, the reinforcing member 80 includes a loop 82 that has been pinched together. FIG. 7I is a plan view of an alternative structure with a reinforcing member 84 that has been integrated into the bend 86, although slits 88 have been formed in the base material. In FIGS. 7J, 7K and 7L, the shape of the open areas 90, 92 in the peak or valley portions 62 have been adjusted to vary the strength at different parts of the stent. Moreover, in FIGS. 7J, 7K and 7L, the reinforcing member 94 has its orientation reversed as compared to the reinforcing members of the peak and valley portions 62 illustrated in the other FIGS. of FIG. 7.

FIG. 9 is a plan view of an alternative embodiment of a stent 42 wherein the pattern of peaks and valleys has been modified to provide multiple side-by-side valley portions 96. Further, interconnecting members 98 are attached at one end thereof to the bends 100 and, at the other end thereof, transition into struts 102.

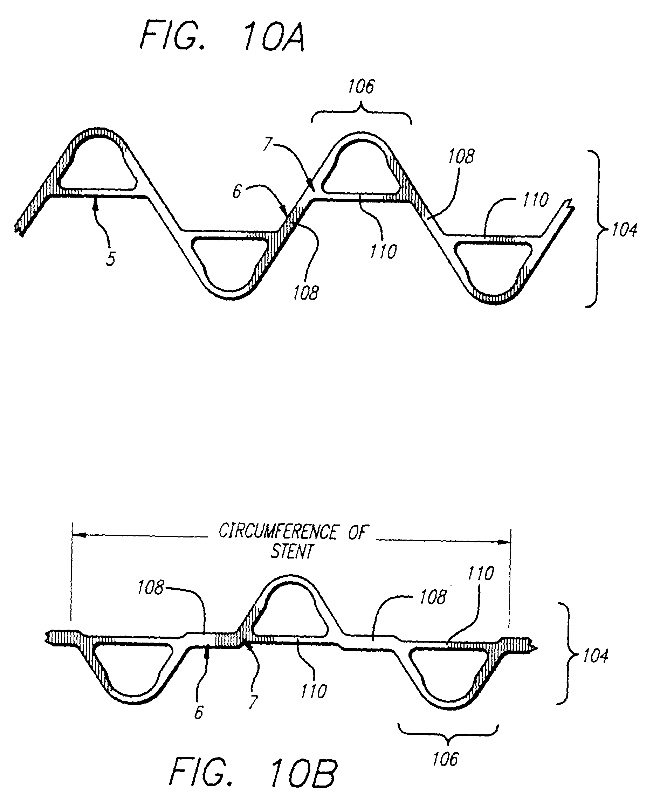

The described stent further includes a bimodal feature as illustrated in FIGS. 10A and 10B. FIG. 10A shows a single cylindrical element 104 having alternating peaks and valleys, wherein each peak and valley is formed by a bend 106 which joins two struts 108. In the conditions shown in FIG. 10A, and as a result of a first stage expansion of the stent, the struts 108 have been slightly bent thereby increasing the circumference of the stent. Thus, the struts 108 are no longer parallel and have spread outwards. The reinforcing member 110 helps maintain the angle formed by the struts 108.

Also, FIG. 10A shows the first mode in which the reinforcing member 110 straightens and locks into position; the loop or kink previously present in the reinforcing member 110 is straightened. The reinforcing member 110 in this configuration provides substantial strength and stiffness to the stent.

In FIG. 10B, the stent has been expanded to a second stage, thereby increasing the circumference of the stent to a greater degree than that which is illustrated in FIG. 10A. As the stent is expanded further, the struts 108 bend at the intersections with the reinforcing members 110 until the struts are aligned with the circumference of the stent as shown in FIG. 10B. At this point, the stent is fully deployed to its maximum diameter. Accordingly, the struts 108 have been pulled straight and are nearly parallel with the reinforcing member 110. In this mode, the stent has reached its maximum circumference, although further increases in the stent conceivably can be achieved by deformation in the struts 108 and the reinforcing member 110. Essentially, the circumference of the stent can be increased by stretching the struts 108 and the reinforcing members 110 further.

It is possible to deploy the stent and reinforcing member with or without two distinct modes. This behavior is controlled by the force required to bend the struts 108 at the intersection of each strut with a reinforcing member 110, as compared to the force required to bend and open the loop in the reinforcing member 110. The behavior can be controlled by the relative widths and lengths of the various structures.

The tubing may be made of suitable biocompatible material such as stainless steel, titanium, tantalum, super-elastic nickel-titanium (NiTi) alloys and even high strength thermoplastic polymers. The stent diameter is very small, so the tubing from which it is made necessarily also must have a small diameter. For PCTA applications, and as an example only, typically the stent has an outer diameter on the order of about 0.165 cm (0.065 in.) in the unexpanded condition, the same outer diameter that characterizes the tubing from which it is made, and can be expanded to an outer diameter of about 0.508 cm (0.200 in.) or more. The wall thickness of the tubing is about 0.008 cm (0.003 in.). For stents implanted in other body lumens, such as in non-coronary PTA applications, the dimensions of the tubing forming the stent correspondingly are larger. The dimensions of the stent will vary depending upon the application and body lumen diameter in which the stent will be implanted.

In the instance when the stent is made from plastic, it may have to be heated within the arterial site where the stent is intended to be deployed so as to facilitate the expansion of the stent. Once expanded, it would then be cooled to retain its expanded state. The stent conveniently may be heated by heating the fluid within the balloon or the balloon directly by a known method. The stent by heating also may be made of materials such as super-elastic NiTi alloys. In this case the stent would be formed full size but deformed (e.g., compressed) into a smaller diameter onto the balloon of the delivery catheter to facilitate transfer to a desired intraluminal site. The stress induced by the deformation transforms the stent from a austenite phase to martensite phase and upon release of the force, when the stent reaches the desired intraluminal location, the stent expands due to the transformation back to the austenite phase.

While the invention has been illustrated and described herein in terms of its use as an intravascular stent, it will be apparent to those skilled in the art that the stent can be used in other instances in all vessels in the body. Because the stent of the present invention is capable of expanding of very large diameters while retaining its structural integrity, it particularly is well suited for implantation in almost any vessel where such devices are used. This feature, coupled with limited longitudinal contraction of the stent when it is radially expanded, provides-a highly desirable support member for all vessels in the body. Other modifications and improvements may be made without departing from the scope of the invention.

## Claims

1. A longitudinally flexible stent (40) for implanting in a body lumen and expandable from a contracted condition to an expanded condition, comprising a plurality of adjacent cylindrical elements (48) which are independently expandable in the radial direction, arranged in alignment along a longitudinal stent axis, and formed in a serpentine wave pattern transverse to the longitudinal axis that contains alternating peaks (52) and valleys (54) and that is configured in size and shape so that the cylindrical elements generally expand in a uniform manner around their circumferences during expansion ofthe stent from its contracted condition to its expanded condition, the stent further comprising at least one interconnecting member connecting adjacent cylindrical elements to one another, **characterised in that**:
the stent further comprises
at least one reinforcing member (44) extending across a peak or a valley; and
that the at least one interconnecting member (50) extends between a reinforcing member of and a cylindrical element and a valley of an adjacent cylindrical element.

2. The stent of claim 1, wherein:
the serpentine wave pattern is an irregular serpentine pattern containing varying degrees of curvature (30, 32, 33) in regions of the peaks and valleys adapted so that radial expansion of the adjacent cylindrical elements is generally uniform around their circumferences during expansion of the stent from its contracted condition to its expanded condition.

3. The stent of either of claims 1 or 2, wherein the at least one interconnecting member connects a valley of one cylindrical element with a reinforcing member of an adjacent cylindrical element.

4. The stent of claim 3, wherein the at least one interconnecting member is unitary with the valley of one cylindrical element and the reinforcing member of the adjacent cylindrical element.

5. The stent of either of claims 1 or 2, wherein the at least one reinforcing member is curved opposite to the respective peak or valley across which it extends.

6. The stent of either of claims 1 or 2, wherein the alternating peaks and valleys further are comprised of straight-length struts intersecting at an angle, and wherein the at least one reinforcing member engages a set of the intersecting struts at a bend point.

7. The stent of claim 6, wherein the straight-length struts are elongated.

8. The stent of claim 6, wherein the bend point is a portion of the strut having reduced material to facilitate bending.

9. The stent of either of claims 1 or 2, wherein the at least one reinforcing member is comprised of a first quarter turn that transitions into a half turn, which transitions into a second quarter turn.

10. The stent of either of claims 1 or 2, wherein an intersection of the at least one reinforcing member with a peak or a valley is rounded.

11. The stent of either of claims 1 or 2, wherein an intersection of the at least one reinforcing member with a peak or a valley is angular.

12. The stent of either of claims 1 or 2, wherein the at least one reinforcing member further is comprised of an enlarged area integrated into the peak or valley in which the at least one reinforcing member is disposed.

13. The stent of either of claims 1 or 2, wherein the at least one reinforcing member further is comprised of an enlarged area integrated into the peak or valley in which the at least one reinforcing member is disposed, the enlarged area having slits therethrough.

14. The stent of either of claims 1 or 2, wherein the stent is formed of a biocompatible material selected from the group consisting of stainless steel, tungsten, tantalum, super-elastic NiTi alloys, and thermoplastic polymers.

15. The stent of either of claims 1 or 2, wherein the stent is formed from a single piece of tubing.

16. The stent of either of claims 1 or 2, wherein the stent is coated with a biocompatible coating.

17. The stent of either of claims 1 or 2, wherein, within a single cylindrical element, the serpentine wave pattern includes a sequence containing a peak, a valley, a peak, a valley, a valley, and a peak.

18. A method for constructing a flexible stent (40) for implantation in a body lumen wherein the stent is expandable from a contracted condition to an expanded condition comprising the steps of:
providing a plurality of adjacent cylindrical elements (48) which are independently expandable in the radial direction and arranged in alignment along a longitudinal stent axis;
forming the cylindrical elements in an irregular serpentine wave pattern transverse to the longitudinal axis and containing a plurality of alternating peaks (52) and valleys (54), wherein the irregular serpentine pattern contains varying degrees of curvature (30, 32, 33) in regions of the peaks and valleys adapted so that radial expansion of the adjacent cylindrical elements is generally uniform around the circumferences of the cylindrical elements during expansion of the stent from its contracted condition to its expanded condition;
providing at least one interconnecting member connecting adjacent cylindrical elements to one another; **characterised in**
providing at least one reinforcing member (44) extending across a width of a peak or a valley; and
providing the at least one interconnecting member (50) extending between a reinforcing member of and a cylindrical element and a valley of an adjacent cylindrical element.

## Patentansprüche

1. Längsflexibler Stent (40) zum Implantieren in ein Körperlumen, der von einem kontrahierten Zustand zu einem aufgeweiteten Zustand aufweitbar ist, umfassend eine Mehrzahl benachbarter zylindrischer Elemente (48), die in der radialen Richtung unabhängig aufweitbar sind, die entlang einer Stentlängsachse ausgerichtet angeordnet sind und die in einem zur Längsachse quer angeordneten Schlangenwellenmuster ausgebildet sind, das abwechselnde Gipfel (52) und Täler (54) enthält und das in Größe und Form so konfiguriert ist, dass die zylindrischen Elemente, während der Aufweitung des Stents von seinem kontrahierten Zustand zu seinem aufgeweiteten Zustand, allgemein gleichmäßig um ihre Umfänge herum aufgeweitet werden, wobei der Stent ferner zumindest ein Verbindungsglied aufweist, das benachbarte zylindrische Elemente miteinander verbindet,
**dadurch gekennzeichnet, dass** der Stent ferner zumindest ein Verstärkungsglied (44) aufweist, das sich quer über einen Gipfel oder ein Tal erstreckt; und
dass sich das zumindest eine Verbindungsglied (50) zwischen einem Verstärkungsglied eines Tals eines zylindrischen Elements und einem Tal eines benachbarten zylindrischen Elements erstreckt.

2. Stent nach Anspruch 1, worin:
das Schlangenwellenmuster ein unregelmäßiges Schlangenmuster ist, das veränderliche Krümmungsgrade (30, 32, 33) in Bereichen der Gipfel und Täler enthält, die so ausgelegt sind, dass während der Aufweitung des Stents von seinem kontrahierten Zustand zu seinem aufgeweiteten Zustand die radiale Aufweitung der benachbarten zylindrischen Elemente um ihre Umfänge allgemein gleichmäßig ist.

3. Stent nach Anspruch 1 oder 2, worin zumindest ein Verbindungsglied ein Tal eines zylindrischen Elements mit einem Verstärkungsglied eines benachbarten zylindrischen Elements verbindet.

4. Stent nach Anspruch 3, worin zumindest ein Verbindungsglied mit dem Tal eines zylindrischen Elements und dem Verstärkungsglied des benachbarten zylindrischen Elements einheitlich ist.

5. Stent nach Anspruch 1 oder 2, worin das zumindest eine Verstärkungsglied zu dem jeweiligen Gipfel oder Tal, über das es sich erstreckt, entgegengesetzt gekrümmt ist.

6. Stent nach Anspruch 1 oder 2, worin ferner die abwechselnden Gipfel und Täler aus mit einem Winkel schneidenden geraden Strebenabschnitten bestehen, und worin das zumindest eine Verstärkungsglied an einen Satz der schneidenden Streben an einem Biegepunkt angreift.

7. Stent nach Anspruch 6, worin die geraden Strebenabschnitte langgestreckt sind.

8. Stent nach Anspruch 6, worin der Biegepunkt ein Abschnitt der Strebe mit reduziertem Material ist, um das Biegen zu erleichtern.

9. Stent nach Anspruch 1 oder 2, worin das zumindest eine Verstärkungsglied aus einer ersten Vierteldrehung besteht, die in eine halbe Drehung übergeht, die in eine zweite Vierteldrehung übergeht.

10. Stent nach Anspruch 1 oder 2, worin der Schnittpunkt des zumindest einen Verstärkungsglieds mit einem Gipfel oder einem Tal gerundet ist.

11. Stent nach Anspruch 1 oder 2, worin der Schnittpunkt des zumindest einen Verstärkungsglieds mit einem Gipfel oder einem Tal gewinkelt ist.

12. Stent nach Anspruch 1 oder 2, worin das zumindest eine Verstärkungsglied ferner aus einer vergrößerten Fläche besteht, die in den Gipfel oder das Tal integriert ist, in dem das zumindest eine Verstärkungsglied angeordnet ist.

13. Stent nach Anspruch 1 oder 2, worin das zumindest eine Verstärkungsglied ferner aus einer vergrößerten Fläche besteht, die in den Gipfel oder das Tal integriert ist, in dem das zumindest eine Verstärkungsglied angeordnet ist, wobei die vergrößerte Fläche Durchgangsschlitze aufweist.

14. Stent nach Anspruch 1 oder 2, worin der Stent aus einem biokompatiblen Material gebildet ist, das aus der Gruppe gewählt ist, die aus rostfreiem Stahl, Wolfram, Tantal, superelastischen NiTi-Legierungen und thermoplastischen Polymeren besteht.

15. Stent nach Anspruch 1 oder 2, worin der Stent aus einem einzelnen Rohrstück gebildet ist.

16. Stent nach Anspruch 1 oder 2, worin der Stent mit einer biokompatiblen Beschichtung beschichtet ist.

17. Stent nach Anspruch 1 oder 2, worin innerhalb eines einzelnen zylindrischen Elements das Schlangenwellenmuster eine Aufeinanderfolge enthält, die einen Gipfel, ein Tal, einen Gipfel, ein Tal, ein Tal und einen Gipfel enthält.

18. Verfahren zur Konstruktion eines flexiblen Stents (40) zum Implantieren in ein Körperlumen, worin der Stent von einem kontrahierten Zustand zu einem aufgeweiteten Zustand aufweitbar ist, das die Schritte aufweist:
Vorsehen einer Mehrzahl benachbarter zylindrischer Elemente (48), die in der radialen Richtung unabhängig aufweitbar sind und längs einer Stentlängsachse ausgerichtet angeordnet sind;
Formen der zylindrischen Elemente in einem unregelmäßigen Schlangenwellenmuster, das quer zur Längsachse angeordnet ist und eine Mehrzahl abwechselnder Gipfel (52) und Täler (54) enthält, worin das unregelmäßige Schlangenmuster variierende Krümmungsgrade (30, 32, 33) in Bereichen der Gipfel und Täler enthält, die so ausgelegt sind, dass während der Aufweitung des Stents von seinem kontrahierten Zustand zu seinem aufgeweiteten Zustand die radiale Aufweitung der benachbarten zylindrischen Elemente um die Umfänge der zylindrischen Elemente herum allgemein gleichmäßig ist;
Vorsehen zumindest eines Verbindungsglieds, das benachbarte zylindrische Elemente miteinander verbindet;
**gekennzeichnet durch**
Vorsehen zumindest eines Verstärkungsglieds (44), das sich quer über eine Breite eines Gipfels oder eines Tals erstreckt; und
Vorsehen des zumindest einen Verbindungsglieds (50), das sich zwischen einem Verstärkungsglied eines Tals eines zylindrischen Elements und einem Tal eines benachbarten zylindrischen Elements erstreckt.

## Revendications

1. Endoprothèse vasculaire réglable dans le sens de la longueur (40) en vue d'être implantée dans une lumière du corps et extensible à partir d'une position contractée à une position étirée, comprenant une pluralité d'éléments cylindriques adjacents (48) qui sont indépendamment extensibles dans la direction radiale, positionnés suivant un alignement le long d'un axe longitudinal de l'endoprothèse vasculaire, et formés suivant un schéma d'ondulation en serpentin transversal à l'axe longitudinal qui contient des pics (52) alternant avec des vallées (54) et qui est configurée en taille et en forme de sorte que les éléments cylindriques s'étendent globalement de manière uniforme autour de leur circonférence pendant l'extension de l'endoprothèse vasculaire à partir de sa position contractée à sa position étirée, l'endoprothèse vasculaire comprenant en outre au moins un membre d'interconnexion connectant les éléments cylindriques adjacents les uns aux autres, **caractérisée en ce que** :
l'endoprothèse vasculaire comprend en outre
au moins un membre de renfort (44) s'étendant sur un pic ou une vallée ; et **en ce qu'**au moins le membre d'interconnexion (50) s'étend entre un membre de renfort et un élément cylindrique et une vallée d'un élément cylindrique adjacent.

2. Endoprothèse vasculaire selon la revendication 1, dans laquelle :
le schéma d'ondulation en serpentin est un schéma d'ondulation en serpentin irrégulier présentant des variations de degrés de courbure (30, 32, 33) dans des régions des pics et vallées adaptées de sorte que l'extension radiale des éléments cylindriques adjacents est globalement uniforme autour de leur circonférence pendant l'extension de l'endoprothèse vasculaire à partir de sa position contractée à sa position étirée.

3. Endoprothèse vasculaire selon l'une quelconque des revendications 1 ou 2, dans laquelle au moins le membre d'interconnexion connecte une vallée d'un élément cylindrique à un membre de renfort d'un élément cylindrique adjacent.

4. Endoprothèse vasculaire selon la revendication 3, dans laquelle au moins le membre d'interconnexion est unitaire avec la vallée d'un élément cylindrique et le membre de renfort de l'élément cylindrique adjacent.

5. Endoprothèse vasculaire selon l'une quelconque des revendications 1 ou 2, dans laquelle au moins le membre de renfort est courbé à l'opposé du pic ou de la vallée respective sur lequel ou laquelle il s'étend.

6. Endoprothèse vasculaire selon l'une quelconque des revendications 1 ou 2, dans laquelle les pics alternant avec les vallées sont en outre constitués de supports de longueur rectiligne faisant intersection à un angle, et dans laquelle au moins le membre de renfort engage un ensemble des supports faisant intersection à un point de pliage.

7. Endoprothèse vasculaire selon la revendication 6, dans laquelle les supports de longueur rectiligne sont allongés.

8. Endoprothèse vasculaire selon la revendication 6, dans laquelle le point de pliage est une partie du support présentant une réduction de matériau afin de faciliter le pliage.

9. Endoprothèse vasculaire selon l'une quelconque des revendications 1 ou 2, dans laquelle au moins le membre de renfort est constitué d'un premier quart de tour qui fait transition en un demitour, qui fait transition en un second quart de tour.

10. Endoprothèse vasculaire selon l'une quelconque des revendications 1 ou 2, dans laquelle une intersection du membre de renfort au moins avec un pic ou une vallée est arrondie.

11. Endoprothèse vasculaire selon l'une quelconque des revendications 1 ou 2, dans laquelle une intersection du membre de renfort au moins avec un pic ou une vallée est angulaire.

12. Endoprothèse vasculaire selon l'une quelconque des revendications 1 ou 2, dans laquelle au moins le membre de renfort est en outre constitué d'une zone agrandie intégrée dans le pic ou la vallée dans lequel ou laquelle au moins le membre de renfort est disposé.

13. Endoprothèse vasculaire selon l'une quelconque des revendications 1 ou 2, dans laquelle au moins le membre de renfort est en outre constitué d'une zone agrandie intégrée dans le pic ou la vallée dans lequel ou laquelle au moins le membre de renfort est disposé, la zone agrandie comprenant des fentes à travers celle-ci.

14. Endoprothèse vasculaire selon l'une quelconque des revendications 1 ou 2, dans laquelle l'endoprothèse vasculaire est formée en un matériau biocompatible sélectionné dans le groupe composé d'acier inoxydable, tungstène, tantale, d'alliages de NiTi super-élastiques, et de polymères thermoplastiques.

15. Endoprothèse vasculaire selon l'une quelconque des revendications 1 ou 2, dans laquelle l'endoprothèse vasculaire est formée à partir d'une seule pièce de tube.

16. Endoprothèse vasculaire selon l'une quelconque des revendications 1 ou 2, dans laquelle l'endoprothése vasculaire est revêtue d'un revêtement biocompatible.

17. Endoprothèse vasculaire selon l'une quelconque des revendications 1 ou 2, dans laquelle, à l'intérieur d'un seul élément cylindrique, le schéma d'ondulation en serpentin comprend une séquence contenant un pic, une vallée, un pic, une vallée, une vallée et un pic.

18. Procédé de fabrication d'une endoprothèse vasculaire réglable (40) en vue d'être implantée dans une lumière du corps dans lequel l'endoprothèse vasculaire est extensible à partir d'une position contractée à une position étirée comprenant les étapes consistant à :
fournir une pluralité d'éléments cylindriques adjacents (48) qui sont indépendamment extensibles dans la direction radiale et positionnés suivant un alignement le long d'un axe longitudinal de l'endoprothèse vasculaire ;
disposer des éléments cylindriques suivant un schéma d'ondulation en serpentin irrégulier transversal à l'axe longitudinal et contenant une pluralité de pics (52) alternant avec des vallées (54),
dans lequel le schéma d'ondulation en serpentin irrégulier présente des variations de degrés de courbure (30, 32, 33) dans des régions des pics et des vallées adaptées de sorte que l'extension radiale des éléments cylindriques adjacents est globalement uniforme autour de la circonférence des éléments cylindriques pendant l'extension de l'endoprothèse vasculaire à partir de sa position contractée à sa position étirée ;
fournir au moins un membre d'interconnexion connectant les éléments cylindriques adjacents les uns aux autres ;
**caractérisé par** la fourniture d'au moins un membre de renfort (44) s'étendant sur une largeur d'un pic ou d'une vallée ; et
la fourniture au moins du membre d'interconnexion (50) s'étendant entre un membre de renfort et un élément cylindrique et une vallée d'un élément cylindrique adjacent.
